# EUROPEAN PATENT APPLICATION

(11) **EP 2 266 952 A1**
(43) Date of publication of application: **29.12.2010**
(21) Application number: 10166317.7
(22) Date of filing: 17.06.2010
(51) Int. Cl.: C07C 249/04, C07C 251/34, C07C 251/44

(54) **Process for producing oxime**

(30) Priority: 18.06.2009 JP 2009145154
(71) Applicant: Sumitomo Chemical Company, Limited, Tokyo 104-8260 (JP)
(72) Inventor: Tsujiuchi, Sho, Niihama Ehime (JP); Watanabe, Nobufumi, Niihama-shi Ehime (JP)
(74) Representative: Vossius & Partner

(57) **Abstract**

An object of the present invention is to produce an oxime with a satisfactory yield by an ammoximation reaction of a ketone with a peroxide and ammonia.

Disclosed is a process for producing oxime, which comprises conducting an ammoximation reaction of a ketone with a peroxide and ammonia in the presence of a mesoporous titanosilicate. The peroxide is preferably an organic peroxide. The mesoporous titanosilicate is preferably a HMS type or MCM-41 type menoporous titanosilicate. The ketone is preferably cycloalkanone.

## Description

The present application claims the Paris Convention priority based on Japanese Patent Application No. 2009-145154 filed on June 18, 2009, the entire content of which is incorporated herein by reference.
The present invention relates to a process for producing an oxime by an ammoximation reaction of a ketone. The oxime is useful as a starting material for amides or lactams.

JP 2006-169168 A, JP 2007-1952 A and JP 2007-238541 A describe, as a catalyst for producing an oxime by an ammoximation reaction of a ketone with a peroxide and ammonia, a microporous titanosilicate having a pore diameter of less than 2 nm, such as a MFI type (including TS-1) or MWW type microporous titanosilicate. Among microporous titanosilicates, TS-1 has been known to be used on an industrial scale of production so far.

Under the circumstances, the present inventors have found that a mesoporous titanosilicate having a pore diameter larger than that of a conventional microporous titanosilicate is useful for the ammoximation reaction, and thus the present invention has been completed.

The present invention provides a process for producing an oxime, which comprises conducting an ammoximation reaction of a ketone with a peroxide and ammonia in the presence of a mesoporous titanosilicate.

The present invention provides the following embodiments.
[1] A process for producing an oxime, which comprises reacting a ketone with a peroxide and ammonia in the presence of a mesoporous titanosilicate.
[2] The process according to [1], wherein the peroxide is an organic peroxide.
[3] The process according to [2], wherein the organic peroxide is a hydroperoxide.
[4] The process according to any one of [1] to [3], wherein the mesoporous titanosilicate is a HMS type or MCM-41 type mesoporous titanosilicate.
[5] The process according to any one of [1] to [4], wherein the reaction is conducted by feeding a ketone and ammonia in a reactor in which a solvent, a mesoporous titanosilicate and a peroxide are charged.
[6] The process according to [5], wherein the solvent is a water-soluble organic solvent.
[7] The process according to [5] or [6], wherein the concentration of ammonia in a liquid phase of the reaction mixture in the reaction of the ketone with the peroxide and ammonia is 1% by weight or more based on the liquid phase.
[8] The process according to any one of [1] to [7], wherein the ketone is cycloalkanone, and the oxime is cycloalkanone oxime.
[9] The process according to [8], wherein the cycloalkanone is cyclohexanone, and the cycloalkanone oxime is cyclohexanone oxime.

The ketone as a starting material may be an aliphatic ketone, an alicyclic ketone or an aromatic ketone. As required, two or more kinds thereof may be used. Specific examples of the ketone include dialkyl ketones such as acetone, ethyl methyl ketone and isobutyl methyl ketone; alkyl alkenyl ketones such as mesityl oxide; alkyl aryl ketones such as acetophenone; diaryl ketones such as benzophenone; cycloalkanones such as cyclopentanone, cyclohexanone, cyclooctanone and cyclododecanone; cycloalkenones such as cyclopentenone and cyclohexenone; and the like. Of these ketones, cycloalkanones are preferred in the present invention, and more preferred is cyclohexanone.

The ketone as a starting material may be obtained, for example, by oxidation of an alkane, or oxidation (dehydrogenation) of a secondary alcohol, or hydration and oxidation (dehydrogenation) of an alkene.

Ammonia to be used in the present invention may be in the gas or liquid state, or in a solution of an organic solvent. The amount of ammonia to be used is preferably adjusted so that the concentration of ammonia in a liquid phase of the reaction mixture becomes 1% by weight or more. The adjustment of the concentration of ammonia in the liquid phase of the reaction mixture to or above a predetermined value allows to increase the conversion rate of a ketone as the starting material and the selectivity to an oxime as the target substance, thus making it possible to increase the yield of an oxime as the target substance. The concentration of ammonia is preferably about 1.5% by weight or more, and is usually about 10% by weight or less, and preferably about 5% by weight or less. The amount of ammonia to be used is usually, as a measure, about 1 mol or more, and preferably about 1.5 mol or more, based on 1 mol of the ketone.

A solvent is usually used for the ammoximation reaction in the present invention, and preferably a water-soluble organic solvent. Preferred examples of the solvent are nitriles such as acetonitrile, propionitrile, butyronitrile, isobutyronitrile, trimethylacetonitrile, valeronitrile, isovaleronitrile and benzonitrile; and alcohols such as methyl alcohol, ethyl alcohol, n-propyl alcohol, isopropyl alcohol, n-butyl alcohol, s-butyl alcohol, t-butyl alcohol and t-amyl alcohol, and nitriles or alcohols having 2 or less carbon atoms are more preferred. As required, two or more kinds thereof can also be used. In the present invention, in view of selectivity to an oxime, the lower the moisture content of the liquid phase of the reaction mixture is, the better.

When the solvent is used, the amount thereof is usually from 1 to 500 parts by weight, and preferably from 2 to 300 parts by weight, based on 1 part by weight of a ketone.

A mesoporous titanosilicate is used as a catalyst for the ammoximation reaction in the present invention. An oxime can be obtained with an excellent yield by using such a mesoporous titanosilicate having a pore diameter larger than that of conventionally used microporous titanosilicate such as an MFI type (including TS-1) or MWW type microporous titanosilicate. The term mesoporous titanosilicate as used herein means a mesoporous titanosilicate having a pore diameter of about 2 to 50 nm. Specific examples of the mesoporous titanosilicate include a titanosilicate having a MCM-41 type structure (hereinafter sometimes referred to as Ti-MCM-41) and a titanosilicate having a HMS type structure (hereinafter sometimes referred to as Ti-HMS).

The mesoporous titanosilicate contains titanium, silicon and oxygen as elements constituting its framework, and may have the framework substantially made from titanium, silicon and oxygen, or may have the framework containing optional elements such as boron, aluminum, gallium, iron and chromium, in addition to titanium, silicon and oxygen. With or without using a binder, this mesoporous titanosilicate may be used after forming into granules or pellets, or may be used in the state of being supported on a carrier.

The content of titanium in the mesoporous titanosilicate is usually 0.0001 or more, and preferably 0.005 or more, and is usually 1.0 or less, and preferably 0.5 or less, in terms of an atomic ratio to silicon (Ti/Si). When the mesoporous titanosilicate contains elements in addition to titanium, silicon and oxygen, the content of the elements is usually 1.0 or less, and preferably 0.5 or less, in terms of an atomic ratio to silicon. Oxygen can exist corresponding to the content of each element other than oxygen and the oxidation number. Typical composition of such a titanosilicate can be represented by the following formula (silicon being standard (= 1):

SiO₂·xTiO₂·yMₙO_{n/2}

wherein M represents at least one element other than silicon, titanium and oxygen, n represents the oxidation number of the element, x is from 0.0001 to 1.0, and y is from 0 to 1.0.

In the formula, M represents elements other than titanium, silicon and oxygen and examples thereof include boron, aluminum, gallium, iron and chromium.

The mesoporous titanosilicate can be prepared, for example, by mixing a titanium compound, a silicon compound and a structure directing agent (template) in the presence of an acidic compound or a basic compound in an aqueous solvent and aging the mixture to obtain a titanosilicate having a mesoporous structure containing the structure directing agent incorporated therein, and removing the structure directing agent from the titanosilicate.

The structure of the mesoporous titanosilicate can be adjusted according to the kind and amount of the structure directing agent to be used. For example, a quaternary ammonium salt such as cetyltrimethylammonium bromide is used when Ti-MCM-41 is prepared, while a primary amine such as n-dodecylamine is used when Ti-HMS is prepared. Examples of the titanium compound include tetraalkyl orthotitanates such as tetra-n-butyl orthotitanate; peroxytitanate such as tetra-n-butylammonium peroxytitanate; and titanium halides. Examples of the silicon compound include tetraalkyl orthosilicates such as tetraethyl orthosilicate; and silica. Examples of the acidic compound include inorganic acids such as hydrochloric acid; and organic acids such as acetic acid. Examples of the basic compound include inorganic bases such as alkali hydroxide and ammonia; and organic bases such as pyridine. Furthermore, examples of the aqueous solvent include water-soluble organic solvents such as water, methanol, ethanol, propanol and 2-propanol, or mixed solvents of water and the water-soluble organic solvents.

The aging temperature in the aging process is usually from 0 to 200°C, and preferably from 20 to 100°C. The aging time is usually from 0.5 to 170 hours, and preferably from 4 to 72 hours.

The titanosilicate having a mesoporous structure containing the structure directing agent incorporated therein can be obtained by the aging process, and then the structure directing agent is removed from the titanosilicate. Examples of the method of removing the structure directing agent include a method of washing with an organic solvent such as methanol, acetone or toluene, a method of washing with hydrochloric acid (an aqueous solution of hydrogen chloride), an aqueous sulfuric acid solution or an aqueous nitric acid solution, and a method of heat-treating at 200 to 800°C. Of these removal methods, any one of methods may be employed, or two or more methods may be employed in combination.

Ti-MCM-41 can be prepared, for example, in accordance with the method described in Microporous and Mesoporous Materials, 2007, pp.312-321, and Ti-HMS can be prepared, for example, in accordance with the method described in Nature, 1994, pp.321-323.

The amount of the mesoporous titanosilicate to be used for the ammoximation reaction may be from about 0.1 to 10% by weight based on the total amount of the reaction mixture.

Examples of the peroxide used in the present invention include hydrogen peroxide and an organic peroxide. In particular, when the organic peroxide is used, the present invention operates more effectively. In addition, when the organic peroxide is used for the ammoximation reaction, the organic peroxide is converted into an alcohol or carboxylic acid. However, it becomes advantageous in view of the cost because the alcohol and carboxylic acid can be recovered by distillation or extraction.

Examples of the organic peroxide as used herein include hydroperoxides such as t-butyl hydroperoxide, cumen hydroperoxide, cyclohexyl hydroperoxide, diisopropylbenzene hydroperoxide, p-menthane hydroperoxide and 1,1,3,3-tetramethylbutyl hydroperoxide; dialkyl peroxides such as t-butylcumyl peroxide, di-t-butyl peroxide, di-t-hexyl peroxide, dicumyl peroxide, α,α'-di(t-butylperoxy)diisopropylbenzene, 2,5-dimethyl-2,5-di(t-butylperoxy)hexane and 2,5-dimethyl-2,5-bis(t-butylperoxy)hexyne-3; peroxy esters such as cumyl peroxyneodacanoate, 1,1,3,3-tetramethylbutyl peroxyneodacanoate, t-hexyl peroxyneodacanoate, t-butyl peroxyneodacanoate, t-butyl peroxyneoheptanoate, t-hexylperoxyvalate, t-butyl peroxypivalate, 2,5-dimethyl-2,5-di(2-ethylhexanoylperoxy)hexane, 1,1,3,3-tetramethylbutylperoxy-2-ethyl hexanoate, t-hexyl peroxy-2-ethylhexanoate, t-butyl peroxy-2-ethylhexanoate, t-butyl peroxylaurate, t-butyl peroxy-3,5,5-trimethylhexanoate, t-hexyl peroxyisopropyl monocarbonate, t-butyl peroxy-2-ethylhexyl monocarbonate, 2,5-dimethyl-2,5-di(benzoylperoxy)hexane, t-butylperoxy acetate, t-hexyl peroxybenzoate and t-butyl peroxybenzoate; diacyl peroxides such as diisobutyryl peroxide, di(3,5,5-trimethylhexanoyl) peroxide, dilauroyl peroxide, disuccinic acid peroxide, dibenzoyl peroxide and di(4-methylbenzoyl)peroxide; and peroxydicarbonates such as diisopropyl peroxydicarbonate, din-propyl peroxydicarbonate, bis(4-t-butylcyclohexyl)peroxydicarbonate, di-2-ethylhexyl peroxydicarbonate and di-sec-butyl peroxydicarbonate. Of these organic peroxides, hydroperoxides are preferred.

The amount of the peroxide to be used is usually from 0.5 to 20 mol, and preferably from 0.5 to 10 mol, based on 1 mol of the ketone.

The ammoximation reaction may be conducted by a batch operation or a continuous operation. The reaction is preferably conducted by a continuous operation in which a liquid phase of the resulting reaction mixture is withdrawn while feeding reaction starting materials in view of productivity and operability.

The continuous reaction can be suitably conducted, for example, by preparing a reaction mixture containing a titanosilicate suspended therein in a reactor, and withdrawing a liquid phase of the resulting reaction mixture through a filter out of the reactor while feeding reaction starting materials such as a ketone. The reactor is preferably a glass lined vessel or a stainless steel vessel in view of preventing the hydrogen peroxide from decomposing.

It is more preferred that a ketone and ammonia are fed into the reactor in which a solvent, a mesoporous titanosilicate and a peroxide are charged in advance. Specifically, first, a solvent, a mesoporous titanosilicate and a peroxide are introduced into the reactor. There is no particular limitation on the order of introduction. After introducing them in the reactor, the mesoporous titanosilicate is suspended by stirring and then a ketone and ammonia are fed. The ketone and ammonia may be fed (co-fed) alone, or a mixture thereof may be fed. After charging a peroxide and a portion of ammonia in the reactor in advance, a ketone and remaining ammonia may be fed into the reactor. Alternatively, after charging a peroxide in the reactor n advance, a peroxide may be additionally added together with a ketone and ammonia.

The reaction temperature of the ammoximation reaction is usually from 50 to 200°C, and preferably from 80°C to 150°C. The reaction pressure may be a normal pressure. In order to make ammonia easily dissolve in a liquid phase of the reaction mixture, the reaction is usually conducted under pressurized conditions of a pressure of 0.2 to 1 MPa in terms of an absolute pressure, and preferably 0.2 to 0.5 MPa. In this case, the pressure may be adjusted using an inert gas such as nitrogen or helium.

The post treatment procedure of the resultant reaction mixture is appropriately selected. For example, the post treatment procedure can be conducted by separating the titanosilicate from the reaction mixture by filtration or decantation, and distilling the liquid phase to separate the obtained oxime.

### EXAMPLES

The present invention will be described by way of the following Examples and Comparative Examples, but it is not construed to limit the present invention thereto. In the following Examples, the liquid phase of the reaction mixture was analyzed by gas chromatography, and the conversion rate of cyclohexane as well as the selectivity and yield of cyclohexanone oxime were calculated based on the results of the analysis.

### Example 1

In a 1 L autoclave (reactor), 154.6 g of an acetonitrile solution containing 3.0% by weight of ammonia, 7.6 g of a cumen solution containing 80% by weight of cumen hydroperoxide, and 2.5 g of Ti-MCM-41 were charged and a vapor phase portion in the reactor was replaced by nitrogen. After the reactor was sealed, the temperature in the reactor was raised to 120°C under stirring. The pressure in the reactor was 0.5 MPa. Next, 10 g of an acetonitrile solution containing 4.7% by weight of cyclohexanone, 115 g of an acetonitrile solution containing 3.8% by weight of ammonia, and 2.6% by weight of cumen hydroperoxide each were fed (co-fed) to the reactor over 1 hour. The concentration of ammonia in the liquid phase of the reaction mixture changed within a range from 1.0 to 3.0% by weight relative to the liquid phase.
After the co-feeding, the liquid phase of the reaction mixture was withdrawn and analyzed by gas chromatography. One hour after the reaction, the conversion rate of cyclohexanone was 94.1%, the selectivity to cyclohexanone oxime was 74.9% and the yield of cyclohexanone oxime was 70.5%. The selectivity to cyclohexanoneimine (a compound produced by imination of cyclohexanone) and impurities derived from the imine, based on the consumed cyclohexanone, was 23.6%.

### Example 2

In a 1 L autoclave (reactor), 155.0 g of an acetonitrile solution containing 3.3% by weight of ammonia, 7.6 g of a cumen solution containing 80% by weight of cumen hydroperoxide, and 2.5 g of Ti-HMS were charged and a vapor phase portion in the reactor was replaced by nitrogen. After the reactor was sealed, the temperature in the reactor was raised to 120°C under stirring. The pressure in the reactor was 0.5 MPa. Next, 10 g of an acetonitrile solution containing 4.7% by weight of cyclohexanone, 115 g of an acetonitrile solution containing 3.8% by weight of ammonia, and 2.6% by weight of cumen hydroperoxide each were fed (co-fed) to the reactor over 1 hour. The concentration of ammonia in the liquid phase of the reaction mixture changed within a range from 1.2 to 3.3% by weight relative to the liquid phase.
After the co-feeding, the liquid phase of the reaction mixture was withdrawn and analyzed by gas chromatography.
One hour after the reaction, the conversion rate of cyclohexanone was 94.8%, the selectivity to cyclohexanone oxime was 98.9% and the yield of cyclohexanone oxime was 93.8%. The selectivity to cyclohexanoneimine (a compound produced by imination of cyclohexanone) and impurities derived from the imine, based on the consumed cyclohexanone, was 1.0%.

### Comparative Example 1

In a 1 L autoclave (reactor), 155.0 g of an acetonitrile solution containing 3.1% by weight of ammonia, 7.6 g of a cumen solution containing 80% by weight of cumen hydroperoxide, and 2.5 g of Ti-MWW (prepared by the same manner as described in Chemistry Letters, 2000, pp. 774-775) were charged and a vapor phase portion in the reactor was replaced by nitrogen. After the reactor was sealed, the temperature in the reactor was raised to 120°C under stirring. The pressure in the reactor was 0.50 MPa. Next, 10 g of an acetonitrile solution containing 4.7% by weight of cyclohexanone, 115 g of an acetonitrile solution containing 3.8% by weight of ammonia, and 2.6% by weight of cumen hydroperoxide each were fed (co-fed) to the reactor over 1 hour. The concentration of ammonia in the liquid phase of the reaction mixture changed within a range from 1.0 to 3.1% by weight relative to the liquid phase.
After the co-feeding, the liquid phase of the reaction mixture was withdrawn and analyzed by gas chromatography. One hour after the reaction, the conversion rate of cyclohexanone was 67.8%, the selectivity to cyclohexanone oxime was 89.8% and the yield of cyclohexanone oxime was 60.9%. The selectivity to cyclohexanoneimine (a compound produced by imination of cyclohexanone) and impurities derived from the imine, based on the consumed cyclohexanone, was 6.9%.

## Claims

1. A process for producing an oxime, which comprises reacting a ketone with a peroxide and ammonia in the presence of a mesoporous titanosilicate.

2. The process according to claim 1, wherein the peroxide is an organic peroxide.

3. The process according to claim 2, wherein the organic peroxide is a hydroperoxide.

4. The process according to any one of claims 1 to 3, wherein the mesoporous titanosilicate is a HMS type or MCM-41 type mesoporous titanosilicate.

5. The process according to any one of claims 1 to 3, wherein the reaction is conducted by feeding a ketone and ammonia in a reactor in which a solvent, a mesoporous titanosilicate and a peroxide are charged.

6. The process according to claim 5, wherein the solvent is a water-soluble organic solvent.

7. The process according to claim 5, wherein the concentration of ammonia in a liquid phase of the reaction mixture in the reaction of the ketone with the peroxide and ammonia is 1% by weight or more based on the liquid phase.

8. The process according to any one of claims 1 to 7, wherein the ketone is cycloalkanone, and the oxime is cycloalkanone oxime.

9. The process according to claim 8, wherein the cycloalkanone is cyclohexanone, and the cycloalkanone oxime is cyclohexanone oxime.
